# EUROPEAN PATENT APPLICATION

(11) **EP 4 374 816 A1**
(43) Date of publication of application: **29.05.2024**
(21) Application number: 22846198.4
(22) Date of filing: 19.07.2022
(51) Int. Cl.: A61C 9/00, A61B 5/00, A61B 1/24, A61C 19/04, G06T 1/00, G06T 17/00, G16H 50/50, G16H 30/00, H04W 4/80

(54) **WIRELESS SCANNING SYSTEM AND WIRELESS SCANNING METHOD**

(30) Priority: 19.07.2021 KR 20210094205
(71) Applicant: Medit Corp., Seoul 07207 (KR)
(72) Inventor: KIM, Hyung Suk, Seoul 02842 (KR)
(74) Representative: MFG Patentanwälte Meyer-Wildhagen Meggle-Freund Gerhard PartG mbB
(86) International application number: PCT/KR2022/010545
(87) International publication number: WO 2023/003328

(57) **Abstract**

A wireless scanning system according to the present disclosure comprises: a wireless scanner which scans a subject to acquire image data; a communication hub which receives the image data transmitted from the wireless scanner; a processing device which is connected to the communication hub and displays the image data received by the communication hub; and a pairing means which is disposed in at least one of the wireless scanner or the communication hub and is configured to connect the wireless scanner and the communication hub to each other.

## Description

### TECHNICAL FIELD

The present disclosure relates to a wireless scanning system and a wireless scanning method.

### BACKGROUND

Recently, a three-dimensional scanner used to measure and analyze a small area such as the inside of an oral cavity has been actively developed. The three-dimensional scanner is configured to enable a user (representatively, a dentist) to easily scan a narrow area.

In this case, the three-dimensional scanner may be electric-communicatively connected to a processor for analyzing a scanned result. In general, the three-dimensional scanner is wiredly connected to the processor. The wired connection enables stable data communication and/or power supply but the scan activity for the user of the three-dimensional scanner can be restricted due to the length of a wire connecting the three-dimensional scanner and the processor with each other.

Accordingly, recently, to overcome the above-described restriction on the use of wired connection, a wireless three-dimensional scanner has been gradually developed and distributed.
The wireless three-dimensional scanner can enhance the degree of freedom of scanning for the user, and the user can obtain more detailed scan data of an object.

However, the wireless three-dimensional scanner needs to be communicatively connected to the processor, and somewhat complex process needs to be performed to connect the processor and the wireless three-dimensional scanner. In addition, when multiple wireless three-dimensional scanners and/or multiple processors are used, communicatively connecting the respective wireless scanners and processors may be more complex.

Meanwhile, in order to transmit or receive a large sized image and control signals between the wireless three-dimensional scanner and the processor, a separate commination hub is required. In addition, the communication hub needs to be paired with the wireless three-dimensional scanner to prevent erroneous transmission of data when multiple wireless three-dimensional scanners are used.

Accordingly, a wireless scanning system and a wireless scanning method capable of performing wireless scanning while simplifying communication connection is required in related industries.

### SUMMARY

To solve the above-described problems, the present disclosure provides a wireless scanning system including a communication hub for receiving image data transmitted from a wireless scanner, and a pairing means for connecting the wireless scanner and the communication hub.

In addition, the present disclosure provides a wireless scanning method for obtaining a three-dimensional model by smoothly transmitting image data obtained by a wireless scanner by using the wireless scanning system to a communication hub.

The technical problems of the present disclosure are not limited to the above-mentioned technical problems, and other technical problems that are not mentioned can be clearly understood by those skilled in the art from the description below.

To solve the above-described problems, a wireless scanning system according to the present disclosure includes a wireless scanner for acquiring image data by scanning an object, a communication hub for receiving the image data transmitted from the wireless scanner, a processing device which is connected to the communication hub and displays the image data received by the communication hub, and a pairing means which is disposed in at least one of the wireless scanner or the communication hub and connects the wireless scanner and the communication hub.

In addition, a wireless scanning system according to the present disclosure may further include more elements in addition to the above-described elements.

In addition, a wireless scanning method according to the present disclosure may perform a process of acquiring a three-dimensional model, including a series of operations, by using the above-described wireless scanning system.

By using a wireless scanning system and a wireless scanning method according to the present disclosure, a wireless scanner can be paired to accurately correspond to a communication hub, the wireless scanner can transmit acquired image data to the communication hub, and a processing device can generate a three-dimensional model on the basis of the image data transferred from the communication hub.

In addition, compared to the conventional method of performing pairing through complex configuration of a device or inputting a PIN number, pairing between a wireless scanner and a communication hub can be performed through a simple operation.

### BRIEF DESCRIPTION OF DRAWINGS

FIGS. 1A, 1B, and 1C illustrate layout structures of a pairing means and a pairing sensor in a wireless scanning system according to the present disclosure.
FIGS. 2 to 5 illustrate a process in which a wireless scanner and a communication hub are paired with each other in a wireless scanning system according to a first embodiment of the present disclosure.
FIG. 6 illustrates a wireless scanning system according to a second embodiment of the present disclosure.
FIG. 7 illustrates a wireless scanning system according to a third embodiment of the present disclosure.
FIG. 8 illustrates a wireless scanning system according to a fourth embodiment of the present disclosure.
FIG. 9 is a flow chart of a wireless scanning method according to the present disclosure.

### <DESCRIPTION OF REFERENCE NUMERALS OF DRAWINGS>

1: Wireless scanning system, 10: Wireless scanner
20: Communication hub, 30: Processing device
40: Pairing means, 50: Pairing sensor
S110: Performing sensing
S120: Exchanging unique information
S130: Performing pairing
S140: Transmitting image data
S150: Generating three-dimensional model
S160: Performing displaying

### DETAILED DESCRIPTION

Hereinafter, some embodiments of the present disclosure will be described in detail with reference to the exemplary drawings. In adding reference numerals to elements of each of the drawings, it should be noted that although the same elements are illustrated in other drawings, like reference numerals may refer to like elements. In addition, for convenience of description, a scale of each of elements illustrated in the accompanying drawings differs from a real scale, and thus, is not limited to a scale illustrated in the drawings. In addition, in describing embodiments of the present disclosure, when it is determined that the detailed description of a related well-known configuration or function may obscure the gist of the embodiments of the present disclosure, the detailed description thereof will be omitted.

In describing elements of embodiments of the present disclosure, terms such as "first," "second," "A," "B," "(a)," and "(b)" may be used. Such terms are merely used to distinguish one element from other elements, and the essence of a corresponding element, the order thereof, the sequence thereof, or the like is not limited by the terms. In addition, unless defined otherwise, all terms including technical and scientific terms used herein have the same meaning as commonly understood by those who are skilled in the art to which the present disclosure belongs. Terms, such as those defined in commonly used dictionaries, should be interpreted as having a meaning consistent with the meaning in the context of the related art, and should not be interpreted in an ideal or excessively formal meaning unless clearly defined herein.

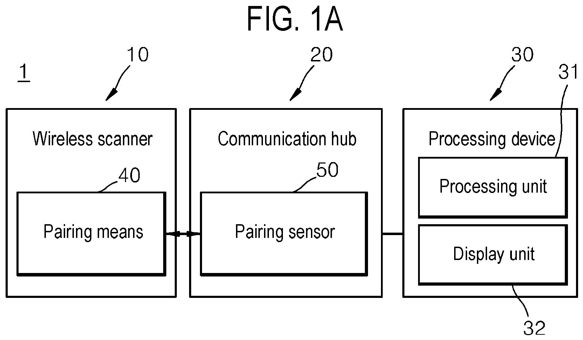

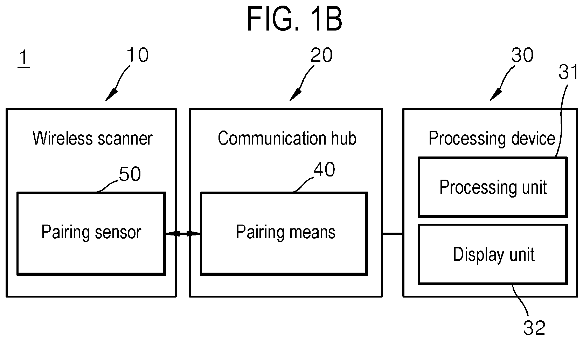

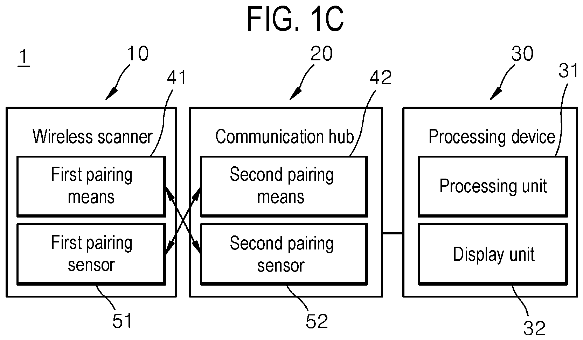

FIGS. 1A, 1B, and 1C illustrate layout structures of a pairing means 40 and a pairing sensor 50 in a wireless scanning system 1 according to the present disclosure.

Referring generally to FIGS. 1A, 1B, and 1C, a wireless scanning system 1 according to the present disclosure includes a wireless scanner 10, a communication hub 20, a processing device 30, and a pairing means 40. The wireless scanner 10 may scan an object to acquire image data indicating the object. In this case, the object may mean an object to be analyzed, and may be an actual oral cavity of a patient or an oral cavity model obtained by impression-taking of the actual oral cavity in the art to which the wireless scanning system according to the present disclosure belongs. The oral cavity model may be formed of a plaster material, but is not limited thereto. Light reflected from a surface of the object is received inside the wireless scanner 10, and the wireless scanner 10 may receive the light through an embedded camera and acquire image data on the basis of the light. The image data may be two-dimensional data, but is not limited thereto.

In addition, the communication hub 20 may receive image data transmitted from the wireless scanner 10. For example, the communication hub 20 may be spaced apart from the wireless scanner 10 by a predetermined distance. The communication hub 20 may be paired with the wireless scanner 10, and may maintain a communicatively connected state. In addition, the communication hub 20 may transmit image data to the processing device 30 described below.

In addition, the processing device 30 may be connected to the communication hub 20. The communication hub 20 may be wiredly or wirelessly connected to the processing device 30. The communication hub 20 may be wiredly connected to a processing unit 31 of the processing device 30 through a data transmission means such as a USB cable, or may be connected to the processing unit through a wireless signal. For example, the communication hub 20 may be wiredly connected to the processing device 30 through a data cable (for example, a USB cable) so that the communication hub 20 stably transmits, to the processing device 30, image data received from the wireless scanner 10 by using wireless communication.

Meanwhile, the processing unit 31 of the processing device 30 may generate a three-dimensional model on the basis of the image data transferred through the communication hub 20. The processing unit 31 may be a microprocessor for performing logical operation, but is not limited thereto, and may be any means capable of generating a three-dimensional model on the basis of the image data. In addition, the display unit 32 of the processing device 30 may display the three-dimensional model generated by the processing unit 31. The display unit 32 may be any element capable of visually displaying the three-dimensional model, and for example, a display device such as a monitor, a tablet PC, and a touch screen may be used.

Hereinafter, the pairing means 40 of the wireless scanning system 1 according to the present disclosure is described.

The wireless communication scanning system 1 according to the present disclosure includes the pairing means 40. The pairing means 40 is disposed in at least one of the wireless scanner 10 and the communication hub 20. The pairing means 40 may communicatively connect the wireless scanner 10 and the communication hub 20. Meanwhile, the pairing means 40 may be sensed by a pairing sensor 50, and the wireless scanner 10 and the communication hub 20 may be connected as a pair through sensing of the pairing means 40 by the pairing sensor 50. When two elements that are spaced apart from each other are paired, it is advantageous in that data can be stably transmitted or received without interference of signals of other elements.

Hereinafter, a layout relationship between the pairing means 40 and the pairing sensor 50 is described.

Referring to FIG. 1A, in a wireless scanning system 1 according to the present disclosure, a pairing means 40 may be included in a wireless scanner 10. For example, the pairing means 40 may be formed to be embedded in the wireless scanner 10, or may be formed at a part of an outer surface of the wireless scanner 10. In this case, for pairing between the wireless scanner 10 and the communication hub 20, the pairing sensor 50 may be formed at the side of the communication hub 20. The pairing sensor 50 may be also formed at a part of an outer surface of the communication hub 20, or may be formed to be embedded in the communication hub 20 according to the type of the sensor.

Referring to FIG. 1B, a pairing means 40 may be included in a communication hub 20. In this case, a pairing sensor 50 for sensing the pairing means 40 may include a wireless scanner 10. That is, in a case where each of the pairing means 40 and the pairing sensor 50 is provided at the wireless scanning system 1 according to the present disclosure, when the pairing means 40 is included in one of the wireless scanner 10 and the communication hub 20, the pairing sensor 50 is included the other one.

Referring to FIG. 1C, two or more pairing means 40 and two or more pairing sensors 50 may be included. For example, the pairing means 40 may include a first pairing means 41 and a second pairing means 42. In this case, the first pairing means 41 may be included in the wireless scanner 10, and the second pairing means 42 may be included in the communication hub 20. In addition, the pairing sensor 50 may include a first pairing sensor 51 and a second pairing sensor 52, the first pairing sensor 51 may be included in the wireless scanner 10, and the second pairing sensor 52 may be included in the communication hub 20. The first pairing means 41 may be paired with the second pairing sensor 52, and the second pairing means 42 may be paired with the first pairing sensor 51. The use of the first pairing means 41 and the second pairing means 42 enables the wireless scanner 10 and the communication hub 20 to be more stably connected.

Hereinafter, the pairing means 40 and the pairing sensor 50 used in the wireless scanning system 1 according to the present disclosure, and a process of communicatively connecting the wireless scanner 10 and the communication hub 20 through the pairing means 40 and the pairing sensor 50 are described in more detail.

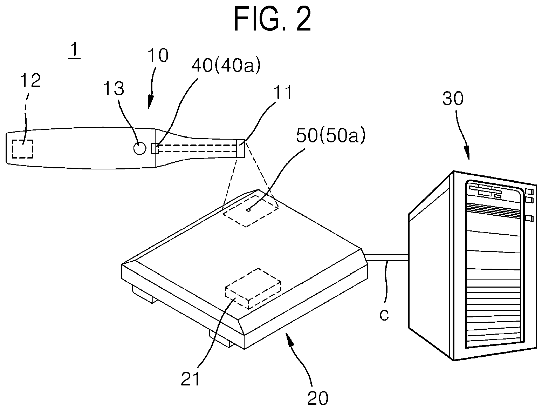

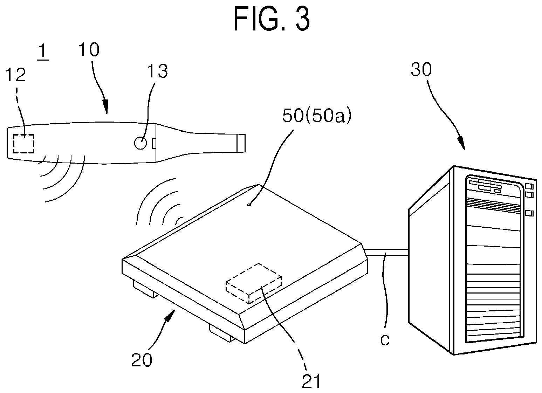

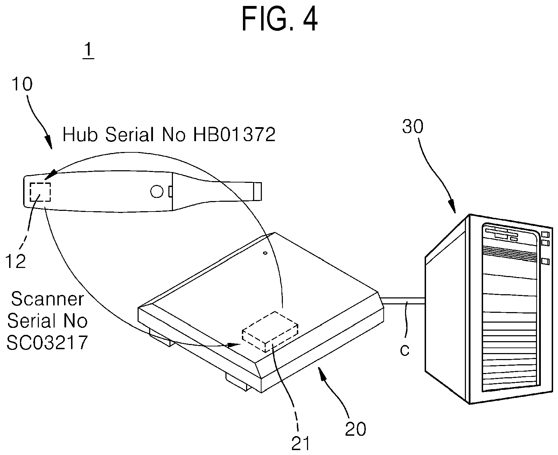

FIGS. 2 to 5 illustrate a process in which a wireless scanner 10 and a communication hub 20 are paired with each other in a wireless scanning system 1 according to a first embodiment of the present disclosure. More specifically, FIG. 2 illustrates a process of sensing a pairing means 40 by a pairing sensor 50, FIG. 3 illustrates a process of sensing a signal by a communication module 12 at the side of a wireless scanner of a wireless scanner 10 and a communication module 21 at the side of a communication hub, and FIG. 4 illustrates a process in which the wireless scanner 10 and the communication hub 20 exchange unique information with each other.

Referring to FIG. 2, in a wireless scanning system 1 according to the present disclosure, the pairing means 40 may be a light projector 40a for emitting a predetermined form of light to from the inside of the wireless scanner 10 to the outside of the wireless scanner 10. The light processor 40a may emit pre-configured light to the outside of the wireless scanner 10, and the form of the light emitted from the light projector 40a may mean a light color, a light pattern, a light brightness, etc. The light emitted from the light projector 40a may be emitted to the outside of the wireless scanner 10 through a light path change member 11 formed inside the wireless scanner 10. More specifically, the light emitted from the light projector 40a may be emitted toward an object by being refracted and/or reflected through the light path change member 11. In addition, the light path change member 11 may allow the light reflected from a surface of the object to be also received inside the wireless scanner 10.

To enable the wireless scanner 10 and the communication hub 20 to be communicatively connected to each other, the wireless scanner 10 may enter into a "pairing mode." The "pairing mode" is contrasted with a "scan mode," and the wireless scanner 10 having entered into the "pairing mode" may emit light having a form distinguished from that of light emitted toward an object, so as to acquire image data of the object in the "scan mode." For example, the light projector 40a may emit one red light, one green light, and multiple blue lights. More specifically, in the "scan mode," the light projector 40a may emit, toward the object, the red light for one unit time, emit the green light for one unit time, and emit the blue light for N unit time. In this case, N may be an integer equal to or greater than 2. The wireless scanner 10 may emit each of the red light, green light, and blue light for one unit time to acquire a color of the object, and may emit the blue light for (N-1) unit time to acquire the shape of the object. However, the present disclosure is not necessarily limited thereto, and the wireless scanner may obtain the color and the shape of the object by emitting a white light by combining the red light, the green light, and the blue light. In addition, the wireless scanner 10 may emit a structure light having at least one pattern toward the object to acquire the shape of the object.

For example, when the wireless scanner 10 enters into the "pairing mode," the light projector 40a may emit light having one color. For example, in the pairing mode, the light projector 40a may emit the red light for a predetermined time. However, the present disclosure is not necessarily limited thereto, and in the pairing mode, the light projector 40a may alternately emit two or more lights for a predetermined time. In another example, in the pairing mode, the light projector 40a may emit the red light while adjusting the brightness for a predetermined time. More specifically, the light projector 40a may emit the red light while gradually increasing the brightness for a predetermined time.

Meanwhile, when the pairing means 40 is the light projector 40a, the light emitted from the light projector 40a may be sensed by the pairing sensor 50. In this case, the pairing sensor 50 may be an illuminance sensor 50a. The illuminance sensor 50a may sense light. More specifically, the illuminance sensor 50a may sense the amount of a specific light, and sense a change in the amount of light. For example, when the amount of sensed light is equal to or greater than a predetermined threshold value, the illuminance sensor 50a may sense a pairing attempt of the wireless scanner 10 subject to pairing. In another example, when a change rate of the amount of sensed light (that is, an average increase rate of the amount of light per unit time or an average decrease rate of the amount of light per unit time) is equal to or greater than a predetermined threshold value, the illuminance sensor 50a may sense a pairing attempt of the wireless scanner 10.

In another example, the illuminance sensor 50a may sense a change in the light color. For example, the light projector 40a may alternately emit the red light and the green light at predetermined time intervals in the pairing mode. In this case, the red light may be (255, 0, 0) with reference to an RGB model, and the green light may be (0, 255, 0) with reference to the RGB model. The illuminance sensor 50a may sense light emission of the light projector 40a, and may sense a pairing attempt of the wireless scanner 10 subject to pairing by sensing whether the light is changed from the red light to the green light or the light is changed from the green light to the red light. Through the above-described scheme, the illuminance sensor 50a may sense the pairing attempt of the wireless scanner 10 so as to prevent light emitted from a light source (for example, room lighting) other than light emitted from the light projector 40a from being erroneously sensed as light emitted from the wireless scanner 10.

Referring to FIG. 3, when the illuminance sensor 50a senses light of the light projector 40a, the wireless scanner 10 and the communication hub 20 may be found by each other through communication modules 12 and 21 for data communication, which are included in the wireless scanner and the communication hub, respectively. For example, the wireless scanner 10 may include a communication module 12 at the side of the wireless scanner for data communication with the outside, and the communication hub 20 may include a communication module 21 at the side of the communication hub for data communication with the outside. After the illuminance sensor 50a senses the light of the light projector 40a, the communication module 12 at the side of the wireless scanner may search for the communication module 21 at the side of the communication hub, and the communication module 21 at the side of the communication hub may search for the communication module 12 at the side of the wireless scanner. The communication module 12 at the side of the wireless scanner and the communication module 21 at the side of the communication hub may transmit predetermined signals for pairing therebetween.

Referring to FIG. 4, in the wireless scanning system 1 according to the present disclosure, the wireless scanner 10 and the communication hub 20 may exchange unique information through the communication modules 12 and 21. For example, the communication modules 12 and 21 may exchange unique information of the wireless scanner 10 and unique information of the communication hub 20 so as to cause the wireless scanner 10 and the communication hub 20 to be paired with each other. More specifically, the communication module 12 at the side of the wireless scanner may transmit its own unique information to the communication module 21 at the side of the communication hub, and the communication module 21 at the side of the communication hub may receive unique information of the wireless scanner 10. In addition, the communication module 21 at the side of the communication hub may transmit its own unique information to the communication module 12 at the side of the wireless scanner, and the communication module 12 at the side of the wireless scanner may receive unique information of the communication hub 20. In this case, the unique information may be a serial number which can identify each of the wireless scanner 10 and the communication hub 20. As such, the wireless scanner 10 and the communication hub 20 may be one-to-one paired through exchange of unique information that each of the wireless scanner 10 and the communication hub 20 has.

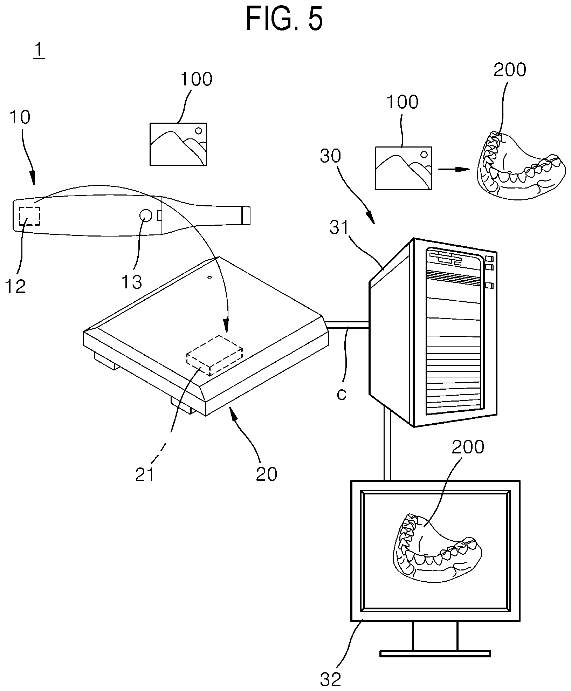

Referring to FIG. 5, after the wireless scanner 10 and the communication hub 20 are one-to-one paired, the wireless scanner 10 may be operated in a "scan mode," to acquire data (for example, image data) indicating an object. The data acquired by the wireless scanner 10 may be transmitted to the communication hub 20. For example, the wireless scanner 10 may transmit image data 100 acquired by scanning the object to the communication hub 20. In this case, the image data 100 is transmitted through the communication module 12 at the side of the wireless scanner, and received by the communication module 21 at the side of the communication hub. The image data 100 may be a two-dimensional plane image, but is not limited thereto.

The communication hub 20 may transfer the image data 100 received from the wireless scanner 10 to the processing device 30 connected through a communication cable c. The processing device 30 may generate a three-dimensional model 200 of the object on the basis of the image data 100 transmitted from the communication hub 20. More specifically, the communication hub 20 may transfer the image data 100 to the processing unit 31 of the processing device 30.

To generate the three-dimensional model 200 on the basis of the image data 100, depth information of the image data 100 may be acquired by using a structure light emitted to the object from the above-described light projector 40a. The wireless scanner 10 and the communication hub 20 may transmit or receive the image data 100 in an environment in which the wireless scanner and the communication hub are one-to-one paired and interference with another device is minimized, and the communication hub 20 may stably generate the three-dimensional model 200 by transmitting the image data 100 to the processing device 30. In addition, the processing device 30 may display at least one of the image data 100 and the three-dimensional model 200 generated on the basis of the image data. More specifically, at least one of the image data 100 and the three-dimensional model 200 may be displayed on a display unit 32 of the processing device 30. The three-dimensional model 200 may be expanded, reduced, or rotated by being displayed on the display unit 32, and a user can apply orthodontic simulation, a prosthetic treatment material, etc. to a patient's oral cavity through the three-dimensional model 200.

In the description above, the light projector 40a may emit a predetermined light pattern from the inside of the wireless scanner 10 to the outside of the wireless scanner 10. That is, a specific pattern may be assigned to the light emitted from the light projector 40a through a pattern generation unit (not shown) additionally formed on a light emission path of the light projector 40a. The pattern generation unit is an element for generating a pattern to emit light having a pattern to the surface of the object so that a three-dimensional model can be generated on the basis of light received by a camera 120 described below. For example, the pattern generation unit may be a pattern mask, and may be a digital micromirror device (DMD). Meanwhile, the predetermined light pattern may have a different form from a light pattern emitted by the light projector 40a when the wireless scanner 10 is in the scan mode. For example, the pattern generation unit may generate a first set of patterns and allow the patterns to be emitted toward the object when the wireless scanner 10 is in the scan mode, and may generate a second set of patterns different from the first set and allow the patterns to be emitted when the wireless scanner 10 is in the pairing mode. The first set of patterns and the second set of patterns may be formed differently in terms of the form, order, etc. of the pattern.

In addition, the pairing sensor 50 disposed in the communication hub 20 may be a pattern sensor for sensing a light pattern emitted to the outside of the wireless scanner 10 by the light projector 40a. For example, a pattern sensor may be a pattern reader capable of sensing a pattern, and the pattern reader may be a camera. The pattern sensor may sense a light pattern emitted by the light projector 40a to sense a pairing attempt of the wireless scanner 10, and the wireless scanner 10 and the communication hub 20 may be paired with each other through the same unique information exchange process as the description made above. The pattern sensor may be formed on an outer surface of the communication hub 20 to easily sense the light pattern emitted from the light projector 40a.

Hereinafter, a wireless scanning system 1 according to a second embodiment of the present disclosure is described.

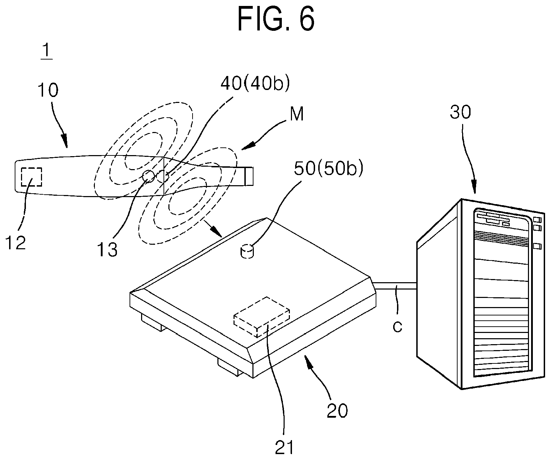

FIG. 6 illustrates a wireless scanning system 1 according to a second embodiment of the present disclosure.

Referring to FIG. 6, a wireless scanning system 1 according to the second embodiment of the present disclosure may use a magnetic unit 40b as a pairing means 40. The magnetic unit 40b may be an element for generating a predetermined magnetic field M, and the magnetic field M generated by the magnetic unit 40b may be constant or variable. The magnetic unit 40b may be a solid magnet such as a permanent magnet, or may be a conductor for generating the magnetic field M when a current is applied.

For example, it is assumed that the magnetic unit 40b is included in the wireless scanner 10. The magnetic unit 40b may be disposed on a lower front surface of the wireless scanner 10 or at one distal upper part of the wireless scanner 10 to enable the wireless scanner 10 and the communication hub 20 to easily be close to each other. The communication hub 20 may include a magnetic sensor 50b configured to sense the magnetic field M generated from the magnetic unit 40b. Meanwhile, the magnetic unit 40b may be included in the communication hub 20. In this case, the magnetic sensor 50b may be included in the wireless scanner 10.

The magnetic sensor 50b may cause the wireless scanner 10 and the communication hub 20 to be paired with each other on the basis of a change in the magnetic field M generated by the magnetic unit 40b. When it is assumed that the magnetic unit 40b is included in the wireless scanner 10, a user may allow the wireless scanner 10 including the magnetic unit 40b to approach the communication hub 20. As the wireless scanner 10 approaches the communication hub 20, the magnetic sensor 50b included in the communication hub 20 may sense a magnetic field M which gradually increases upon approaching of the magnetic unit 40b. Accordingly, the magnetic sensor 50b may sense a pairing attempt of the wireless scanner 10. The magnetic sensor 50b may be a Hall sensor or an MR sensor, but is not necessarily limited to the type herein.

After the magnetic sensor 50b senses the pairing attempt of the wireless scanner 10, the communication module 12 at the side of the wireless scanner and the communication module 21 at the side of the communication hub may be paired through exchanging of unique information. The paired wireless scanner 10 and the communication hub 20 may transmit or receive image data 100 in an environment in which interference with another device is minimized, and the communication hub 20 may stably generate a three-dimensional model 200 by transmitting the image data 100 to the processing device 30.

Hereinafter, a wireless scanning system 1 according to the third embodiment of the present disclosure is descried.

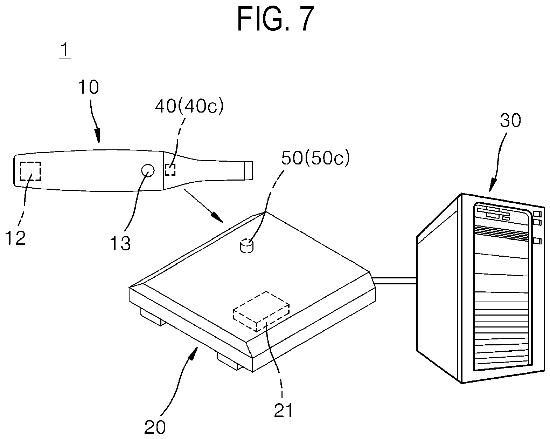

FIG. 7 illustrates a wireless scanning system 1 according to the third embodiment of the present disclosure.

Referring to FIG. 7, a wireless scanning system 1 according to a third embodiment of the present disclosure may use a near-field communication unit 40c as a pairing means 40. The near-field communication unit 40c may be an element for generating a predetermined RF signal, and an RF signal generated by the near-field communication unit 40c may be constant or variable.

For example, it is assumed that the near-field communication unit 40c is included in the wireless scanner 10. The near-field communication unit 40c may be disposed on a lower front surface of the wireless scanner 10 or at one distal upper part of the wireless scanner 10 to enable the wireless scanner 10 and the communication hub 20 to easily be close to each other. The communication hub 20 may include a near-field communication sensor 50c configured to sense the RF signal generated from the near-field communication unit 40c. Meanwhile, the near-field communication unit 40c may be included in the communication hub 20. In this case, the near-field communication sensor 50c may be included in the wireless scanner 10.

The near-field communication sensor 50c may cause the wireless scanner 10 and the communication hub 20 to be paired with each other on the basis of a change in the RF signal generated by the near-field communication unit 40c. When it is assumed that the near-field communication unit 40c is included in the wireless scanner 10, a user may allow the wireless scanner 10 including the near-field communication unit 40c to approach the communication hub 20. As the wireless scanner 10 approaches the communication hub 20, the near-field communication sensor 50c included in the communication hub 50b may sense an RF signal upon the approaching of near-field communication unit 40c. Accordingly, the near-field communication sensor 50c may sense a pairing attempt of the wireless scanner 10.

After the near-field communication sensor 50c senses the pairing attempt of the wireless scanner 10, the communication module 12 at the side of the wireless scanner and the communication module 21 at the side of the communication hub may be paired through exchanging of unique information. The paired wireless scanner 10 and the communication hub 20 may transmit or receive image data 100 in an environment in which interference with another device is minimized, and the communication hub 20 may stably generate a three-dimensional model 200 by transmitting the image data 100 to the processing device 30.

Hereinafter, a wireless scanning system 1 according to a fourth embodiment of the present disclosure is described.

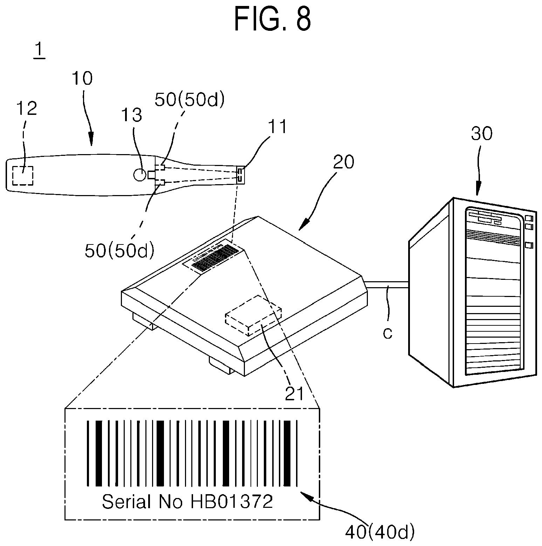

FIG. 8 illustrates a wireless scanning system 1 according to the fourth embodiment of the present disclosure.

Referring to FIG. 8, a wireless scanning system 1 according to the fourth embodiment of the present disclosure may use an identifier 40d as a pairing means 40. The identifier 40d may include unique information of an element to which the identifier 40d is attached. As the identifier 40d, one of a bar code and a QR code may be used, but it is not limited thereto, and any physical element which can include unique information is possible.

The identifier 40d may be formed to be attached to an outer surface of the communication hub 20. For example, the identifier 40d may be attached on the top of the communication hub 20 so as to ease image-capturing, and coating-processing may be performed for the identifier 40d so that damage due to an external factor is prevented.

Meanwhile, the wireless scanner 10 may include a camera 50d as a pairing sensor 50 for sensing the identifier 40d configured as a bar code or a QR code. The camera 50d may receive light reflected from a surface of the identifier 40d and introduced into the wireless scanner 10. The introduced light may include a shape of the bar code or the QR code, and the wireless scanner 10 may acquire an image of the bar code or the QR code. When the image of the bar code or the QR code is acquired, the shape of the image may be sensed, and unique information may be acquired from the image. In this case, the acquired unique information may indicate the type of a device to which the identifier 40d is attached. For example, the identifier 40d may include a 5-digit unique number (a part of unique information) indicating the device to which the identifier 40d is attached. The 5-digit unique information may indicate the communication hub 20. The wireless scanner 10 may store a list of unique numbers indicating the communication hub 20. That is, the wireless scanner 10 may identify whether a unique number acquired by capturing an image of the identifier 40d is included in a pre-stored list of unique numbers. If the unique number acquired by the wireless scanner 10 is included in the pre-stored list of unique numbers, the wireless scanner may sense that the device to which the identifier 40d is attached is the communication hub 20.

According to a need, the above-described camera 50d may be a code reader for receiving light reflected from a surface of the identifier 40d and promptly sensing a code of the identifier 40d, rather than an element of receiving light reflected from a surface of an object. The code reader may easily sense the identifier 40d by emitting IR light, and promptly acquire unique information included in the identifier 40d. Meanwhile, the code reader may not directly emit the IR, and may emit red light to the above-described light projector 40a to sense the identifier 40d.

After the camera 50d senses the identifier 40d, the communication module 12 at the side of the wireless scanner and the communication module 21 at the side of the communication hub may be paired with each other through exchanging of unique information. The paired wireless scanner 10 and the communication hub 20 may transmit or receive image data 100 in an environment in which interference with another device is minimized, and the communication hub 20 may stably generate a three-dimensional model 200 by transmitting the image data 100 to the processing device 30.

Meanwhile, if the pairing between the wireless scanner 10 and the communication hub 20 is successfully completed, the processing device 30 may transmit a control signal to the wireless scanner 10 through the communication module 21 at the side of the communication hub so as to control the wireless scanner 10 to vibrate or flicker for a predetermined time. In addition, if the pairing between the wireless scanner 10 and the communication hub 20 is successfully completed, the processing device 30 may control a status indicator (for example, an LED lamp for displaying the state of the communication hub) included in the communication hub 20 to flicker. In addition, a guide message indicating that pairing has been successfully completed may be displayed on a user interface screen displayed on the display unit 32.

If the pairing means 40 is not sufficiently close to the pairing sensor 50 to sense the pairing sensor, the processing device 30 may also display, through the display unit 32, a guide message inducing a user to move the wireless scanner 10 closer to the communication hub 20. Alternatively, the processing device 30 may display, through the display unit 32, a guide message indicating that search for a device to be paired has failed.

Meanwhile, referring to FIGS. 2 to 8, the wireless scanner 10 may further include a pairing switch 13 which is pressed in one direction and causes a predetermined pairing signal to be generated. A user may control the pairing means 40 to operate by pressing the pairing switch 13 for a predetermined time. When the user presses the pairing switch 13, the light projector 40a may emit light that is emitted in the "pairing mode," and a current may flow through the magnetic unit 40b, which generates the magnetic field M according to application of the current, by the pressing of the pairing switch 13. In addition, the near-field communication unit 40c may generate an RF signal when the user presses the pairing switch 13. The pairing switch 13 may control the camera 50d to operate to sense the identifier 40d.

By using the pairing switch 13, the pairing process may be performed through operation of the pairing means 40 and the pairing sensor 50 when the user presses the pairing switch 13, and the pairing means 40 may be controlled not to operate when the user does not press the pairing switch 13, whereby inaccurate pairing can be prevented. In addition, by controlling a device for generating a pairing signal to be paired with a device including the pairing sensor 50 through pressing of the pairing switch 13, there are advantages in that a complex configuration process can be omitted, after releasing the previously paired connection, pairing can be established with a device for generating a new pairing signal, and prompt pairing switching can be performed.

According to the description made above, by using the wireless scanning system 1 according to the present disclosure, the user can pair the wireless scanner 10 and the communication hub 20 in a simpler and faster manner compared to the conventional art in which pairing is performed through a complex configuration process. Accordingly, user convenience can be enhanced.

Hereinafter, a wireless scanning method according to the present disclosure is described. In describing the wireless scanning method according to the present disclosure, a description that is duplicative of the description of the wireless scanning system above is briefly mentioned or omitted.

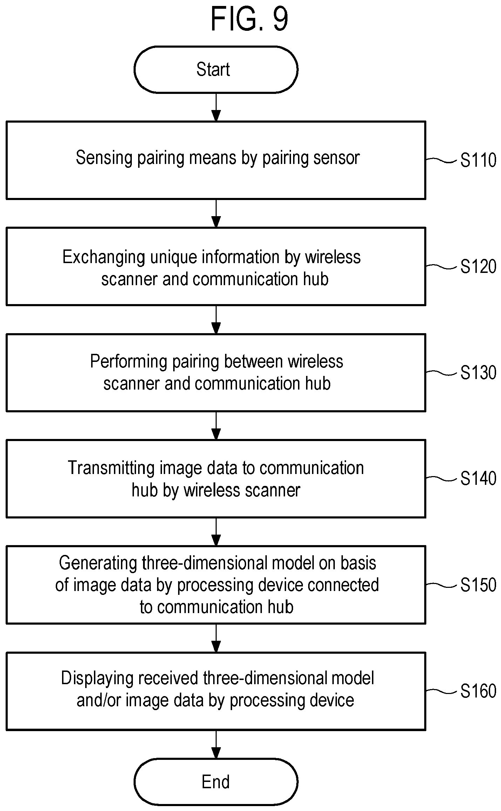

FIG. 9 is a flow chart of a wireless scanning method according to the present disclosure.

Referring to FIG. 9, a wireless scanning method according to the present disclosure includes sensing a pairing means disposed in at least one of a wireless scanner and a communication hub by a pairing sensor disposed in the other one among the wireless scanner and the communication hub, in which the pairing means is not disposed (operation S110). For example, when the pairing means is included in the wireless scanner, the pairing sensor is included in the communication hub. Alternatively, when the pairing means is included in the communication hub, the pairing means is included in the communication hub. When the pairing means is formed as multiple pairing means such as a first pairing means and a second pairing means and the pairing sensor is also formed as multiple pairing sensors such as a first pairing sensor and a second pairing sensor, the first pairing means and the first pairing sensor may be included in the wireless scanner and the second pairing means and the second pairing sensor may be included in the communication hub. The description on the layout relationship between the pairing means and the pairing sensor is identical to the description made above.

As described above, the pairing means may be a light projector capable of emitting light having a predetermined color and/or light having a predetermined pattern, and in this case, the pairing sensor may be an illuminance sensor or a pattern sensor capable of receiving and sensing light emitted from the light projector. When the pairing means is the light projector, the pairing means may be included in the wireless scanner, and in this case, the pairing sensor may be included in the communication hub.

In addition, the pairing means may be a magnetic unit for generating a magnetic field. In this case, the pairing sensor may be a magnetic sensor capable of sensing a magnitude of a magnetic field generated from the magnetic unit and/or a change in the magnitude of the magnetic field. In addition, the pairing means may be a near-field communication unit for generating a predetermined RF signal, and in this case, the pairing sensor may be a near-field communication sensor capable of sensing the RF signal. When the pairing sensor is the magnetic unit or the near-field communication unit, the pairing means may be included in the wireless scanner, and in this case, the pairing sensor may be included in the communication hub. On the other hand, the pairing means may be included in the communication hub, and in this case, the pairing sensor may be included in the wireless scanner. The pairing means included in the wireless scanner and/or the pairing sensor may be disposed on a lower surface or at an upper distal end of the wireless scanner to ease pairing with the communication hub.

In addition, the pairing means may be an identifier attached or engraved on the communication hub, and in this case, the pairing sensor may be a camera which is included in the wireless scanner and receives light introduced into the wireless scanner. The identifier may be one of a bar code and a QR code, and the pairing sensor may sense the communication hub through a unique number (as described above, a part of unique information) obtained by sensing the identifier.

The description of the pairing operation between the pairing means and the pairing sensor is identical to the description made in the wireless scanning system according to the present disclosure, and thus a detailed description thereof is omitted.

Meanwhile, the sensing (operation S110) includes pressing the pairing switch formed on at least one of the wireless scanner and the communication hub to generate a predetermined pairing signal in the pairing means. That is, a user may press the pairing switch to control the pairing means and the pairing sensor to enter into a "pairing mode" in which the pairing process can be performed.

Thereafter, when the pairing sensor senses the pairing means and senses a pairing attempt of the wireless scanner and/or the communication hub, the wireless scanner and the communication hub may exchange unique information of the wireless scanner and unique information of the communication hub through communication modules for data communication, which the wireless scanner and the communication hub have, respectively (operation S120). For example, a communication module at the side of the wireless scanner may transmit its own unique information to the communication hub, and a communication module at the side of the communication hub may transmit its own unique information to the wireless scanner. Accordingly, the communication module at the side of the wireless scanner may receive unique information of the commination hub and the communication module at the side of the communication hub may receive unique information of the wireless scanner. The unique information may be a serial number of each device (wireless scanner and communication hub), but is not limited thereto.

When the unique information of the wireless scanner and the unique information of the communication hub are exchanged, the wireless scanner and the communication hub may be paired one-to-one (operation S130). In this case, data interference by an unpaired device can be minimized, the wireless scanner can stably transmit image data to the communication hub, and the communication hub can also stably receive data.

Through the one-to-one pairing between the wireless scanner and the communication hub, the paired wireless scanner and the communication hub may transmit or receive data. More specifically, the wireless scanner may transmit image data to the communication hub (operation S140). For example, the image data may be two-dimensional plane data obtained by scanning an object (an actual oral cavity of a patient or an oral cavity model manufactured by impression-taking of the oral cavity) by the wireless scanner.

Thereafter, the communication hub may transfer the image data received from the wireless scanner to a processing device electrically connected to the communication hub, and the processing device may display the received image data (operation S160). The image data may be displayed on a user interface screen shown on a display unit included in the processing device.

Meanwhile, the processing device may generate a three-dimensional model indicating the object on the basis of the image data transferred from the communication hub (operation S150). Through the generation of the three-dimensional model, the object can be easily analyzed in the processing device, and various oral cavity treatment simulations (orthodontic simulation, prosthetic treatment material, etc.) can be applied without manufacturing multiple oral cavity models.

When the generating of the three-dimensional model (operation S 150) is performed, in the above-described displaying operation (operation S160), the three-dimensional model may be displayed. In this case, the displaying (operation S160) may include displaying only the image data, display only the three-dimensional model, or displaying the image data and the three-dimensional model together. Accordingly, the user can easily identify and analyze image data obtained through scanning of an object and/or a three-dimensional model generated on the basis of the image data.

The above description provides an example of the technical idea of the present disclosure for illustrative purposes only, and those who are skilled in the art to which the present disclosure belongs will appreciate that various modifications and changes are possible without departing from the essential features of the present disclosure.

Therefore, the embodiments disclosed in the present disclosure are intended not to limit but to illustrate the technical idea of the present disclosure, and the scope of the technical idea of the present disclosure is not limited by the embodiments. The scope of the present disclosure should be construed on the basis of the accompanying claims, and all of the technical ideas included within the scope equivalent to the claims should be construed as belonging to the scope of the present disclosure.

### INDUSTRIAL APPLICABILITY

The present disclosure provides a wireless scanning system including a communication hub for receiving image data transmitted from a wireless scanner, and a pairing means for connecting the wireless scanner and the communication hub, and a wireless scanning method for acquiring a three-dimensional model by smoothly transmitting the image data acquired by the wireless scanner by using the wireless scanning system to the communication hub.

## Claims

1. A wireless scanning system comprising:
a wireless scanner configured to scan an object to acquire image data;
a communication hub configured to receive the image data transmitted from the wireless scanner;
a processing device which is connected to the communication hub and is configured to display the image data received by the communication hub; and
a pairing means which is disposed in at least one of the wireless scanner and the communication hub and is configured to connect the wireless scanner or the communication hub to each other.

2. The wireless scanning system of Claim 1, wherein the pairing means is a light projector configured to emit a predetermined form of light from inside of the wireless scanner to outside of the wireless scanner,
the communication hub comprises an illuminance sensor which senses the light, and
the illuminance sensor configured to sense at least one of a brightness of the light and a color change of the light to cause the wireless scanner or the communication hub to be paired with each other.

3. The wireless scanning system of Claim 2, wherein the light projector is configured to emit light having a form different from light emitted when scanning the object.

4. The wireless scanning system of Claim 1, wherein the pair means is a light projector configured to emit a predetermined light pattern from inside of the wireless scanner to outside of the wireless scanner,
wherein the communication hub comprises a pattern sensor which senses the light pattern, and
wherein the pattern sensor is configured to sense the light pattern to cause the wireless scanner and the communication hub to be paired with each other.

5. The wireless scanning system of Claim 1, wherein the pairing means is a magnetic unit configured to generate a predetermined magnetic field,
wherein one of the wireless scanner and the communication hub comprises a magnetic sensor configured to sense the magnetic field generated from the magnetic unit, and
wherein the magnetic sensor causes the wireless scanner and the communication hub to be paired with each other on the basis of a change in the magnetic field generated by the magnetic unit.

6. The wireless scanning system of Claim 1, wherein the pairing means is a near-field communication unit configured to generate a predetermined RF signal,
wherein one of the wireless scanner and the communication hub comprises a near-field communication sensor configured to sense the RF signal generated from the near-field communication unit, and
wherein the near-field communication sensor causes the wireless scanner and the communication hub to be paired with each other on the basis of the RF signal generated by the near-field communication unit.

7. The wireless scanning system of Claim 1, wherein the pairing means is an identifier attached to the communication hub, and
wherein the wireless scanner is configured to acquire an image of the identifier to cause the wireless scanner and the communication hub to be paired with each other.

8. The wireless scanning system of one of Claims 1 to 7, wherein the wireless scanner and the communication hub further comprise communication modules for data communication, respectively, and
wherein the communication modules are configured to exchange unique information of the wireless scanner and unique information of the communication hub with each other to cause the wireless scanner and the communication hub to be paired with each other.

9. The wireless scanning system of Claim 1, wherein the processing device is configured to generate a three-dimensional model of the object on the basis of the image data transmitted from the communication hub, and
wherein the processing device is configured to display at least one of the image data or the three-dimensional model.

10. The wireless scanning system of one of Claims 1 to 7, wherein at least one of the wireless scanner or the communication hub further comprises a pairing switching which is pressed in one direction to generate a predetermined pairing signal.

11. A wireless scanning method comprising:
sensing a pairing means disposed in at least one of a wireless scanner or a communication hub by a pairing sensor disposed in the other one of the wireless scanner and the communication hub, in which no pairing means is disposed;
according to the sensing of the pairing means by the pairing sensor, exchanging unique information of the wireless scanner and unique information of the communication hub with each other, by communication modules which the wireless scanner and the communication hub have, respectively, and performing data communication;
according to the exchanging of the unique information of the wireless scanner and the unique information of the communication hub, causing the wireless scanner and the communication hub to be paired with each other; and
receiving image data transmitted from the wireless scanner by the communication hub and displaying the image data by a processing device connected to the communication hub.

12. The wireless scanning method of Claim 11, wherein the pairing means is a light projector which emits a predetermined form of light from inside of the wireless scanner to outside of the wireless scanner,
wherein the communication hub comprises an illuminance sensor which senses the light, and
wherein the illuminance sensor senses at least one of a brightness of the light or a color change of the light to cause the wireless scanner and the communication hub to be paired with each other.

13. The wireless scanning method of Claim 11, wherein the pair means is a light projector which emits a predetermined light pattern from inside of the wireless scanner to outside of the wireless scanner,
wherein the communication hub comprises a pattern sensor which senses the light pattern, and
wherein the pattern sensor senses the light pattern to cause the wireless scanner and the communication hub to be paired with each other.

14. The wireless scanning method of Claim 11, wherein the pairing means is a magnetic unit which generates a predetermined magnetic field,
wherein one of the wireless scanner and the communication hub comprises a magnetic sensor which senses the magnetic field generated from the magnetic unit, and
wherein the magnetic sensor causes the wireless scanner and the communication hub to be paired with each other on the basis of a change in the magnetic field generated by the magnetic unit.

15. The wireless scanning method of Claim 11, further comprising generating a three-dimensional model by the processing device on the basis of the image data,
wherein the displaying comprises displaying the three-dimensional model.
